# FASCICULE DE BREVET EUROPEEN

(11) **EP 3 220 168 B1**
(45) Date de publication et mention de la délivrance du brevet: **17.08.2022**
(21) Numéro de dépôt: 17160612.2
(22) Date de dépôt: 13.03.2017
(51) Int. Cl.: G01N 15/00, G01N 33/28, F16H 57/04, G01N 15/06, G01V 3/10

(54) **CAPTEUR DE PARTICULES DANS UN FLUIDE D'UN SYSTÈME DE LUBRIFICATION**
PARTIKELSENSOR IN EINEM FLUID EINES SCHMIERSYSTEMS
PARTICLE SENSOR IN A FLUID OF A LUBRICATION SYSTEM

(30) Priorité: 14.03.2016 BE 201605183
(43) Date de publication de la demande: 20.09.2017
(73) Titulaire: SAFRAN AERO BOOSTERS S.A., 4041 Herstal (BE)
(72) Inventeur: ROYEN, Jean-Luc, 4357 Donceel (BE)
(74) Mandataire: Gevers Patents

(56) Documents cités:
- EP-A1- 2 455 774
- WO-A1-2007/088015
- WO-A1-2015/028569
- CN-U- 203 365 280
- JP-A- 2008 008 885
- US-A- 3 748 576
- US-A- 3 952 592
- US-A- 4 100 491
- US-A- 4 785 239
- US-A1- 2009 164 161
- US-A1- 2009 189 464
- US-A1- 2011 089 938
- US-A1- 2015 293 009

## Description

### Domaine technique

Selon un premier aspect, la présente invention concerne un capteur de particules prévu pour détecter des particules dans un fluide d'un système de lubrification, par exemple d'une turbomachine. Selon un deuxième aspect, la présente invention concerne une méthode de détection de particules dans un fluide d'un système de lubrification, par exemple d'une turbomachine.

### Art antérieur

L'usure de pièces lubrifiées par un système de lubrification, par exemple l'usure de paliers d'une turbomachine d'aéronef, génère des particules dans le lubrifiant. La présence de ces particules fournit par conséquent une indication de l'état d'usure des pièces lubrifiées.

Le document FR2443691 A1 divulgue un détecteur de présence de particules dans un milieu fluide. Selon le fonctionnement de ce détecteur connu, des particules s'accumulent de façon à former un pont entre deux électrodes présentes dans le fluide et l'impédance de ce pont est mesurée.

Ce détecteur connu présente plusieurs inconvénients. Premièrement, il faut du temps et une certaine quantité de particules pour que ce pont puisse se former. Deuxièmement, un tel pont peut être détruit par les vibrations se produisant dans un aéronef en vol. Ces vibrations ont donc pour effet de ralentir la détection et d'augmenter le seuil de sensibilité du détecteur connu. A cause des vibrations, le détecteur connu est aussi probablement incapable de fournir une mesure quantitative du nombre de particules présentes dans le fluide car les vibrations peuvent détruire le pont ou faire que des particules s'en détachent.

Le document WO2007/088015 A1 divulgue un capteur pour détecter des particules dans un flux de fluide. Ce capteur connu comporte des bobines qui sont traversées par le flux de fluide.

Un problème de ce capteur connu est que son installation nécessite de modifier fortement le système de lubrification et de manipuler les composants internes du capteur puisque les bobines du capteur doivent être disposées autour d'un conduit du système de lubrification. Un autre problème est que la détection est basée sur l'évolution d'un signal temporel. Par conséquent, d'une part, une mesure instantanée ne permet de savoir avec certitude s'il y a des particules et d'autre part, le traitement des données requis est assez lourd.

Le document EP 2 455 774 A1 décrit un capteur ayant une surface exposée à un fluide et un aimant permanent qui produit un champ magnétique dans une zone de la surface. Un détecteur détecte les particules ferromagnétiques dans la zone de la surface du capteur.

### Résumé de l'invention

Un des buts de l'invention est de fournir un capteur facile à installer sur un système de lubrification et permettant de détecter rapidement des particules sans traitement de données particulier.

En outre, un des buts de l'invention est de fournir un capteur plus rapide, avec un seuil de sensibilité plus bas et avec une meilleure sensibilité que le capteur divulgué dans FR2443691 A1.

A cet effet, selon un premier aspect, l'invention propose un capteur selon la revendication 1.

Le capteur de l'invention permet de détecter des particules magnétiques ou magnétisables. Nous entendons par ces termes des particules dont la perméabilité relative est en module plus grande que 1, de préférence plus grande que 10 et de manière encore plus préférée plus grande que 100 à 25°C.

Le capteur selon l'invention fonctionne grâce à l'influence de particules sur l'unité de mesure, et à la non-influence, ou influence non-mesurable, de ces particules sur l'unité de référence. En particulier, un signal magnétique émis par la première bobine émettrice et capté par la première bobine de réception est influencé par la présence des particules alors qu'un signal magnétique émis par la deuxième bobine émettrice et capté par la deuxième bobine de réception ne l'est pas suffisamment pour que cela soit mesurable. Il n'est donc pas nécessaire qu'un pont de particules se forme pour que ces particules soient détectées. Cela permet de diminuer le seuil de sensibilité et le temps de réponse du capteur selon l'invention par rapport à celui connu de FR2443691 A1.

L'aimant permet d'attirer les particules vers le capteur, en particulier vers l'unité de mesure. Il n'est donc pas nécessaire que le fluide traverse le capteur pour que les particules soient détectées. Le positionnement de l'aimant entre l'unité de mesure et l'unité de référence et à égale distance des deux permet une influence similaire de l'aimant sur l'unité de mesure et l'unité de référence, rendant ainsi possible la mesure différentielle. De plus, ce positionnement permet que l'aimant soit proche du fluide tout en attirant les particules vers l'unité de mesure.

Le fait que l'aimant soit à égale distance de l'unité de mesure et l'unité de référence indique que la première bobine émettrice est à une même distance de l'aimant que la deuxième bobine émettrice et la première bobine de réception est à une même distance de l'aimant que la deuxième bobine de réception

En outre, le capteur selon l'invention permet de mesurer une différence entre un signal provenant de l'unité de référence et de l'unité de mesure. Comme il est plus facile de mesurer une différence que de mesurer une valeur absolue, et ce, en particulier en électronique, le capteur selon l'invention a une sensibilité particulièrement bonne, ce qui lui permet de fournir une mesure quantitative de la quantité de particules dans le fluide, c'est-à-dire du niveau d'encrassement du fluide. Cela lui permet aussi, de détecter, à tout moment et très rapidement, des particules dans le fluide, sans avoir besoin d'un traitement de données particulier.

De plus, le fait que le capteur selon l'invention permette de mesurer une différence entre un signal provenant de l'unité de référence et de l'unité de mesure permet que la détection par le capteur selon l'invention ne soit pas, ou que peu, affectée par des artefacts ou des paramètres extérieurs comme la température. Cela permet aussi de ne pas devoir effectuer d'étalonnage du capteur selon l'invention.

Le fait que les unités de mesure et de référence sont agencées de manière symétrique par rapport à un plan situé entre l'unité de mesure et l'unité de référence indique que l'unité de mesure est l'image par symétrie orthogonale de l'unité de référence dans ce plan. Par conséquent, la première bobine émettrice est à une même distance de ce plan que la deuxième bobine émettrice et la première bobine de réception est à une même distance de ce plan que la deuxième bobine de réception.

Cette symétrie permet qu'un signal électrique émis par la première bobine de réception soit égal à un signal électrique émis par la deuxième bobine de réception en l'absence de particules à proximité de l'unité de mesure. En outre, cette disposition permet que les unités de mesure et de référence puissent être en image miroir l'une de l'autre, l'unité de mesure étant ainsi proche du fluide de lubrification et l'unité de référence étant ainsi plus éloignée de ce fluide. De plus, cette disposition permet au capteur d'être particulièrement facile à fabriquer, compact et solide.

De préférence, l'aimant est aussi symétrique par rapport à ce plan, avec le pôle nord de l'aimant d'un côté du plan et le pôle sud de l'aimant de l'autre côté du plan.

Le capteur selon l'invention est aussi intéressant car il permet une détection en continu des particules dans le fluide, sans devoir arrêter le dispositif lubrifié par le système de lubrification et sans devoir faire d'inspection visuelle. Grâce à cela, si le capteur est installé avec un système de lubrification dans un aéronef, il est possible pour le pilote de l'aéronef de vérifier le niveau d'encrassement du système de lubrification en vol. Il est aussi possible pour une personne au sol de vérifier ce niveau lorsque l'aéronef est en vol ou lorsque l'aéronef est au sol.

De préférence, l'aimant est un aimant permanent, c'est-à-dire un élément passif. Il est intéressant d'avoir un aimant qui est un élément passif car il ne nécessite pas d'alimentation. De manière plus préférée, le capteur ne comprend qu'un seul aimant permanent, ce qui est particulièrement avantageux, notamment car il permet une bonne homogénéité de flux magnétique pour attirer les particules. L'aimant peut être standard.

De préférence, la première bobine émettrice, la première bobine de réception, la deuxième bobine émettrice, la deuxième bobine de réception et l'aimant ont une symétrie circulaire. Cela permet une fabrication de précision et un montage du capteur aisés.

Le capteur comprend une paroi externe de contact prévue pour être en contact avec le fluide et pour empêcher l'entrée du fluide et des particules dans le capteur.

Le fait que cette paroi soit externe permet que le fluide et les particules restent à l'extérieur du capteur. Il est donc facile d'installer le capteur sur un conduit du système de lubrification, par exemple en faisant un trou dans ce conduit, sans devoir modifier profondément le système de lubrification. En outre, le fait que cette paroi soit externe permet que les composants internes du capteur ne doivent pas être modifiés lors de l'installation.

De plus, cette paroi externe empêche la pénétration du fluide, par exemple de l'huile, et des particules à l'intérieur des bobines, en particulier dans les bobines de l'unité de mesure. En effet, la présence de fluide et de particules dans les bobines de l'unité de mesure pourrait perturber la mesure différentielle.

De manière avantageuse, le capteur comprend en outre un premier élément de carcasse ferromagnétique traversant au moins partiellement les premières bobines émettrice et de réception et un deuxième élément de carcasse ferromagnétique traversant au moins partiellement les deuxièmes bobines émettrice et de réception.

La paroi externe de contact peut comprendre une partie de surface du premier élément de carcasse ferromagnétique.

Dans le cadre du présent document, une "carcasse magnétique" est un ensemble des pièces ferromagnétiques canalisant les lignes d'induction d'une machine ou d'un appareil électromagnétique. Elle peut comprendre notamment des éléments en fer doux. Cette disposition permet d'étendre de manière particulièrement efficace la portée de l'aimant afin d'attirer les particules.

Dans une réalisation de l'invention, la première bobine de réception entoure la première bobine émettrice et la deuxième bobine de réception entoure la deuxième bobine émettrice.

Dans une autre réalisation de l'invention, la première bobine émettrice entoure la première bobine de réception et la deuxième bobine émettrice entoure la deuxième bobine de réception.

Préférentiellement, l'aimant est fait dans un matériau comprenant du samarium-cobalt.

Ce matériau permet à l'aimant de garder ses propriétés magnétiques à de hautes températures qui peuvent être atteinte dans un système de lubrification utilisé dans une turbomachine d'un aéronef, par exemple aux alentours de 200°C.

Dans une réalisation de l'invention, le capteur comprend en outre un premier circuit magnétique entourant au moins partiellement ladite unité de mesure pour concentrer des lignes de champ magnétiques générées par ledit aimant.

La paroi externe de contact peut comprendre une partie de surface du premier circuit magnétique.

Préférentiellement, le capteur comprend en outre un deuxième circuit magnétique entourant au moins partiellement ladite unité de référence pour concentrer des lignes de champ magnétiques générées par ledit aimant.

Dans une réalisation de l'invention, les première et deuxième bobines émettrices sont connectées électriquement en parallèle.

Cela permet qu'elles soient alimentées électriquement de manière identique.

Dans une réalisation de l'invention, le capteur comprend en outre un circuit électronique agencé pour :
- envoyer un signal électrique source vers les première et deuxième bobines émettrices, et
- recevoir et amplifier un signal électrique de différence représentatif d'une différence entre une grandeur électrique mesurée aux bornes de la première bobine de réception et une autre mesurée aux bornes de la deuxième bobine de réception.

Dans une réalisation de l'invention, la première bobine émettrice, la première bobine de réception, la deuxième bobine émettrice, la deuxième bobine de réception et l'aimant sont disposés dans un environnement proche du système de lubrification qui peut être soumis à des températures de l'ordre de 200°C et le circuit électronique est disposé de façon plus éloignée par rapport au système de lubrification de façon à ne pas être soumis à des températures aussi hautes.

De préférence, les grandeurs électriques sont des tensions électriques. Elles peuvent aussi être des courants électriques.

Préférentiellement, le signal électrique source comprend des impulsions en courant et/ou en tension.

Des tensions électriques mises en jeu dans le capteur selon l'invention sont préférentiellement entre 100 mV et 12V, préférentiellement entre 0.5 V et 2 V aux bornes de bobines.

En particulier, de façon préférée, le signal électrique source comprend des impulsions dont la durée est inférieure à 20% d'une durée séparant deux impulsions successives.

Un tel rapport ON/OFF de moins de 20% permet de limiter l'échauffement des bobines. Par conséquent, l'amplitude du signal électrique source peut être plus grande qu'avec un rapport ON/OFF plus grand. En outre, cela permet d'atténuer les signaux parasites.

De manière plus préférée, le signal électrique source comprend des impulsions dont la durée est inférieure à 10% d'une durée séparant deux impulsions successives.

Dans une réalisation de l'invention, le signal électrique source a une fréquence comprise entre 50 et 200 Hz.

Une telle fréquence permet de ne pas augmenter le temps de réponse du capteur.

De manière plus préférée, le signal électrique source a une fréquence comprise entre 100 et 150 Hz.

Dans une réalisation de l'invention, le circuit électronique comprend un circuit astable pour créer un signal électrique comprenant des impulsions, un circuit d'inversion pour inverser ledit signal électrique comprenant des impulsions et un amplificateur pour amplifier ledit signal électrique comprenant des impulsions inversé, afin de générer le signal électrique source.

De manière préférée, le circuit électronique comprend un amplificateur opérationnel agencé pour amplifier le signal électrique de différence.

Le capteur comprend au moins un élément de blocage agencé de façon à empêcher le fluide d'entrer en contact avec la première bobine émettrice et de la première bobine de réception.

Si les particules entraient en contact direct avec les bobines, il pourrait être difficile de les en enlever et donc de réutiliser le capteur. L'élément de blocage est de préférence compris dans l'unité de mesure. L'élément de blocage est formé d'une pièce distincte des autres pièces du capteur. Notamment, l'élément de blocage n'est pas d'une seule pièce avec l'aimant, le premier élément de carcasse ferromagnétique, le deuxième élément de carcasse ferromagnétique, le premier circuit magnétique ou le deuxième circuit magnétique. L'élément de blocage peut être fixé avec au moins l'une des autres pièces du capteur. L'élément de blocage peut par exemple être une grille ou une paroi.

L'invention propose en outre un système de lubrification, en particulier de turbomachine, comprenant au moins un capteur selon l'invention. De préférence, le système de lubrification comprend aussi un un conduit comprenant une partie de détection, le capteur étant positionné de façon à ce que l'unité de mesure se trouve entre la partie de détection et l'unité de référence. Cela permet que l'unité de mesure soit plus influencée par les particules que l'unité de référence. Cela permet aussi que l'unité de mesure ait une fonction de blocage qui empêche le fluide de s'approcher de l'unité de référence.

De préférence, le capteur est impénétrable par les particules. En d'autres termes, le capteur ne comporte pas de trou en contact fluidique avec le conduit dans lequel les particules pourraient entrer.

L'invention propose en outre une turbomachine et un aéronef comprenant au moins un tel capteur ou un tel système de lubrification.

Selon un deuxième aspect, l'invention propose une méthode de détection de particules dans un fluide d'un système de lubrification, la méthode comprenant les étapes de:
- fournir un capteur selon l'invention,
- disposer le capteur de façon à ce que des particules présentes dans le fluide puissent influencer une induction magnétique captée par la première bobine de réception,
- envoyer un signal électrique source vers les première et deuxième bobines émettrices,
- amplifier une grandeur électrique de différence entre un signal électrique aux bornes de la première bobine de réception et un signal électrique aux bornes de la deuxième bobine de réception.

La méthode selon l'invention permet de détecter des particules magnétiques ou magnétisables. Nous entendons par ces termes des particules dont la perméabilité relative est en module plus grande que 1, de préférence plus grande que 10 et de manière encore plus préférée plus grande que 100 à 25°C.

L'étape d'envoyer un signal électrique source vers la première et la deuxième bobines émettrice peut comprendre ou consister en appliquer un signal électriques aux bornes des bobines émettrices.

Les avantages mentionnés pour le dispositif s'appliquent mutatis mutandis à la méthode.

### Brève description des figures

D'autres caractéristiques et avantages de l'invention apparaîtront à la lecture de la description détaillée qui suit pour la compréhension de laquelle on se reportera aux figures annexées parmi lesquelles :
- la figure 1 est une vue schématique d'un capteur selon une réalisation de l'invention,
- la figure 2 est une vue schématique d'un capteur selon une réalisation de l'invention, et
- la figure 3 illustre un schéma électronique d'un capteur selon une réalisation de l'invention.

### Modes de réalisation de l'invention

La présente invention est décrite avec des réalisations particulières et des références à des figures mais l'invention n'est pas limitée par celles-ci. Les dessins ou figures décrits ne sont que schématiques et ne sont pas limitants.

Dans le contexte du présent document, les termes « premier » et « deuxième » servent uniquement à différencier les différents éléments et n'impliquent pas d'ordre entre ces éléments.

Sur les figures, les éléments identiques ou analogues peuvent porter les mêmes références.

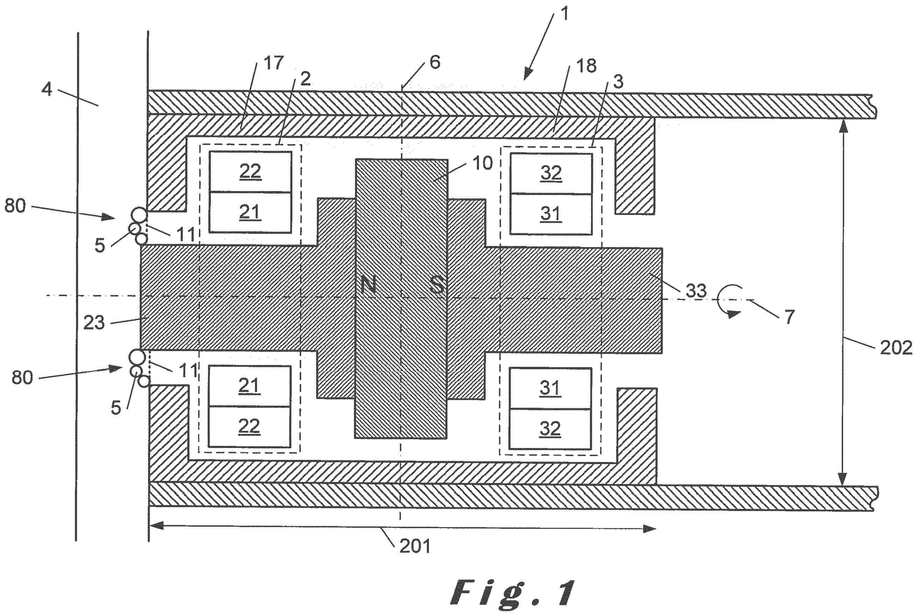

La figure 1 est une vue schématique d'un capteur 1 selon une réalisation possible de l'invention.

Le capteur 1 comprend une unité de mesure 2. L'unité de mesure 2 comprend une première bobine émettrice 21 et une première bobine de réception 22 coaxiale avec la première bobine émettrice 21 et couplée magnétiquement à la première bobine émettrice 21. Le capteur 1 comprend une unité de référence 3. L'unité de référence 3 comprend une deuxième bobine émettrice 31 et une deuxième bobine de réception 32 coaxiale avec la deuxième bobine émettrice 31 et couplée magnétiquement à la deuxième bobine émettrice 31. La deuxième bobine émettrice 31 est identique à la première bobine émettrice 21, et la deuxième bobine de réception 32 est identique à la première bobine de réception 21.

Dans la réalisation de l'invention illustrée à la figure 1, la première bobine de réception 22 entoure la première bobine émettrice 21 et la deuxième bobine de réception 32 entoure la deuxième bobine émettrice 31. Il est aussi possible, tout en restant dans le cadre de l'invention, que la première bobine émettrice 21 entoure la première bobine de réception 22 et la deuxième bobine émettrice 31 entoure la deuxième bobine de réception 32.

Les unités de mesure 2 et de référence 3 sont préférentiellement de de symétrie cylindrique avec un même axe de symétrie cylindrique 7. Le capteur 1 peut aussi être de symétrie cylindrique autour de cet axe 7.

L'unité de mesure 2 est prévue pour être disposée suffisamment près d'un fluide circulant dans un conduit 4 d'un système de lubrification, de façon à ce qu'une induction magnétique émise par la première bobine émettrice 21 et captée par la première bobine de réception 22 puisse être influencée par des particules 5 présentes dans ce conduit 4. En particulier, l'unité de mesure 2 peut être mise en contact avec le fluide. L'unité de référence 3 est prévue pour être suffisamment loin du conduit 4 du système de lubrification pour que des particules 5 présentes dans ce conduit 4 ne puissent pas influencer de façon mesurable une induction magnétique captée par la deuxième bobine de réception 32. L'unité de mesure 2 du capteur 1 est située entre le conduit 4 et l'unité de référence 3.

Le conduit 4 comprend de préférence une partie de détection 80 prévue pour être en contact avec l'unité de mesure 2 du capteur 1, de façon à ce que l'unité de mesure 2 soit entre la partie de détection 80 et l'unité de référence.

Les inductions magnétiques émises par la première bobine émettrice 21 et la deuxième bobine émettrice 31 sont préférentiellement de 1 à 10 mT.

L'unité de référence 3 est symétrique avec l'unité de mesure 2 par symétrie orthogonale par rapport à un plan 6 disposé entre l'unité de mesure 2 et l'unité de référence 3. Le plan 6 est perpendiculaire à l'axe de symétrie 7.

Le fluide, et donc les particules 5 qu'il contient, sont bloqués par un élément de blocage 11 afin qu'il ne rentre pas dans l'unité de mesure 2 en elle-même, et en particulier qu'il n'entre pas en contact avec la première bobine émettrice 21 et la première bobine de réception 22. Il peut y avoir une pluralité d'éléments de blocage 11. L'élément de blocage 11 est de préférence situé entre la partie de détection 80 et l'unité de mesure 2. De préférence, l'élément de blocage 11 n'est pas formé par une partie d'une autre pièce du capteur 1, mais est une pièce distincte.

Le capteur 1 selon l'invention comprend un aimant 10 pour générer un champ magnétique continu, agencé de façon à attirer des particules 5 du fluide vers l'unité de mesure 2. L'aimant 10 est disposé entre l'unité de mesure 2 et l'unité de référence 3, de façon à influencer autant l'une que l'autre. L'aimant 10 est préférentiellement un aimant permanent, plus préférentiellement un aimant permanent comprenant du samarium-cobalt. L'attraction générée par l'aimant 10 permet que les particules 5 soient particulièrement présentes dans une région où elles puissent influencer une induction magnétique émise par la première bobine émettrice 21 et captée par la première bobine de réception 22. De préférence, l'aimant 10 permet de générer une induction magnétique continue comprise entre 10 et 100 mT. L'aimant 10 a préférentiellement son axe nord-sud le long de l'axe de symétrie 7.

Le capteur 1 comprend en outre préférentiellement un premier élément de carcasse ferromagnétique 23 traversant au moins partiellement les premières bobines émettrice 21 et de réception 22 et un deuxième élément de carcasse ferromagnétique 33 traversant au moins partiellement les deuxièmes bobines émettrice 31 et de réception 32.

Dans la réalisation de l'invention montrée à la figure 1, le capteur 1 comprend en outre un premier circuit magnétique 17 entourant au moins partiellement l'unité de mesure 2 pour concentrer des lignes de champ magnétiques générées par l'aimant 10. Le premier élément de carcasse ferromagnétique 23 dépasse préférentiellement le premier circuit magnétique 17 en direction du conduit 4 du système de lubrification.

En outre, dans la réalisation de l'invention montrée à la figure 1, le capteur 1 comprend un deuxième circuit magnétique 18 entourant au moins partiellement l'unité de référence 3 pour concentrer des lignes de champ magnétiques générées par l'aimant 10. Le deuxième élément de carcasse ferromagnétique 33 dépasse préférentiellement le deuxième circuit magnétique 18 dans la direction opposée au conduit 4 du système de lubrification.

Il est préféré que l'unité de mesure 2 et l'unité de référence 3 soient fixées ensemble à moins de 30 mm l'une de l'autre, plus préférentiellement moins de 20 mm l'une de l'autre. Cela permet d'avoir l'unité de référence 3 et l'unité de mesure 2 dans un espace compact. En outre, cela permet que des influences extérieures, telle que la température, soient prises en compte de la même façon par l'unité de mesure 2 et l'unité de référence 3.

Par exemple, une longueur 201 du capteur 1 peut être de 25 à 30 mm. En outre, par exemple un diamètre 202 du capteur 1 peut être de 15 à 25 mm.

Dans une réalisation de l'invention, l'espace entre les premières bobines émettrice 21 et de réception 22, le premier élément de carcasse ferromagnétique 23 et l'aimant 10, ainsi que l'espace entre les deuxièmes bobines émettrice 31 et de réception 32, le deuxième élément de carcasse ferromagnétique 33 et l'aimant 10 comprend du polyétheréthercétone (PEEK). Préférentiellement, ces espaces en sont remplis. Le polyétheréthercétone permet de supporter les premières bobines émettrice 21 et de réception 22, le premier élément de carcasse ferromagnétique 23, les deuxièmes bobines émettrice 31 et de réception 32, le deuxième élément de carcasse ferromagnétique 33 et l'aimant 10.

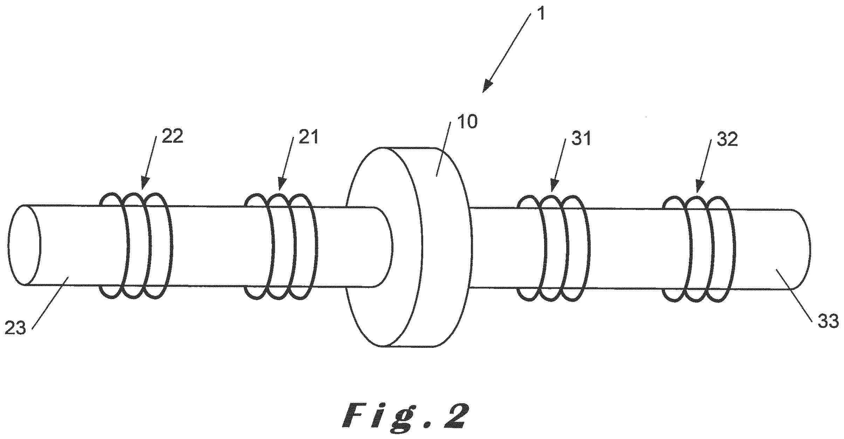

La figure 2 est une vue schématique en perspective d'un capteur 1 selon une autre réalisation de l'invention. Dans la réalisation de l'invention illustrée à la figure 2, la première bobine de réception 22 est disposée à côté de la première bobine émettrice 21 le long du premier élément de carcasse ferromagnétique 23 et la deuxième bobine de réception 32 est disposée à côté de la deuxième bobine émettrice 31 le long du deuxième élément de carcasse ferromagnétique 33.

Dans une réalisation de l'invention non-illustrée, la première bobine de réception 22 et la première bobine émettrice 21 sont enroulées ensemble et la deuxième bobine de réception 32 et la deuxième bobine émettrice 31 sont enroulées ensemble.

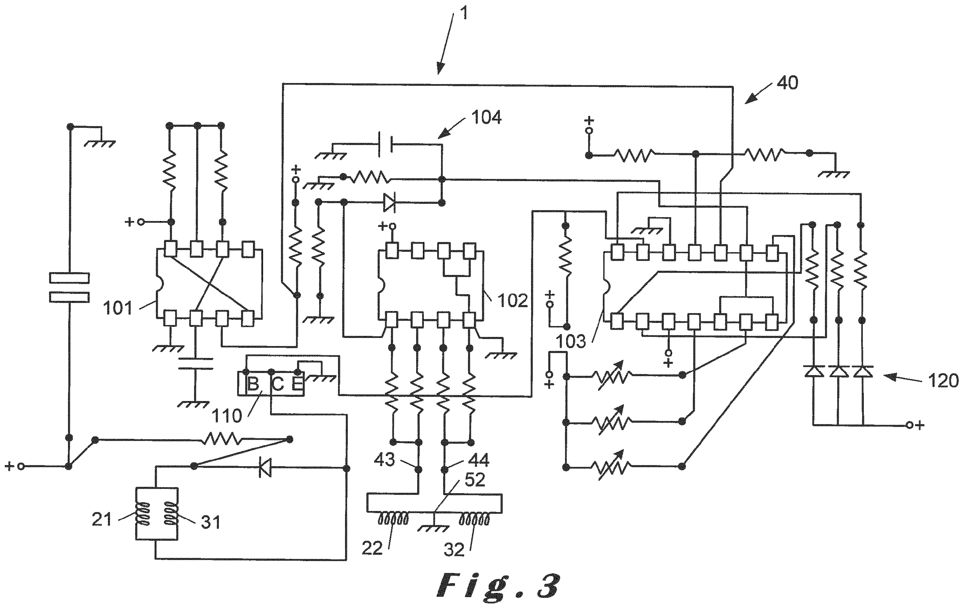

La figure 3 illustre un schéma électronique du capteur 1 dans une réalisation de l'invention.

Le capteur 1 comprend en effet, préférentiellement, un circuit électronique 40 agencé pour envoyer un signal électrique source vers les première 21 et deuxième 31 bobines émettrices, et recevoir et amplifier un signal électrique de différence représentatif d'une différence entre une grandeur électrique mesurée aux bornes de la première bobine de réception 22 et une autre grandeur électrique mesurée aux bornes de la deuxième bobine de réception 32.

La première bobine émettrice 21 et la deuxième bobine émettrice 31 sont de préférence connectées en parallèle l'une avec l'autre.

Le signal électrique source est préférentiellement un signal pulsé, comprenant, plus préférentiellement, des impulsions dont la durée est inférieure à 20% d'une durée séparant deux impulsions successives, et, encore plus préférentiellement, des impulsions dont la durée est inférieure à 10% d'une durée séparant deux impulsions successives.

La fréquence du signal électrique source est préférentiellement comprise entre 50 et 200 Hz, plus préférentiellement comprise en 100 et 150Hz. Elle peut par exemple être de 120 Hz.

Le signal électrique source est préférentiellement généré, dans le circuit électronique 40, par un circuit astable 101, par exemple un NE555, pour créer un signal électrique comprenant des impulsions, suivi par un circuit d'inversion pour inverser ledit signal électrique comprenant des impulsions, le circuit d'inversion faisant par exemple partie d'un quadruple comparateur différentiel 103 de type 399, lui-même suivi par un amplificateur 110, par exemple un amplificateur Darlington.

Un côté de la première bobine de réception 22 et un côté de la deuxième bobine de réception 32 sont connectés ensemble en un point 52 qui est lui-même connecté à la terre. L'autre côté de la première bobine de réception 22 est connecté à une connexion 43 du circuit électronique 40 et l'autre côté de la deuxième bobine de réception 32 est connecté à une connexion 44 du circuit électronique 40. Les connexions 43 et 44 sont connectées, dans le circuit électronique 40, à un amplificateur, par exemple un amplificateur opérationnel 102, comme un amplificateur opérationnel à faible puissance 272, ayant une fonction de circuit récepteur et amplificateur.

Un ensemble 104 formé d'une diode, un condensateur et une résistance de décharge a pour fonction de transformer le signal de sortie de l'amplificateur opérationnel 102 en signal analogique qui est utilisé dans le quadruple comparateur différentiel 103.

Le circuit électronique 40 peut afficher une indication de la quantité de particules, par exemple par des LEDs 120, ou transmettre, par communication avec fil ou sans fil, cette indication à un organe extérieur, comme à un ordinateur présent dans un aéronef sur un système de lubrification duquel le capteur 1 est installé.

En d'autres termes, l'invention se rapporte à un capteur 1 de particules prévu pour détecter des particules dans un conduit 4 d'un système de lubrification. Le capteur 1 comprend une unité de mesure 2, agencée pour être disposée en contact avec un fluide circulant dans le conduit 4, et une unité de référence 3, agencée pour être disposée hors contact avec le fluide circulant dans le conduit 4. Une détection des particules est réalisée en comparant des signaux électriques provenant de l'unité de mesure 2 et de l'unité de référence 3.

La présente invention a été décrite en relation avec des modes de réalisations spécifiques, qui ont une valeur purement illustrative et ne doivent pas être considérés comme limitatifs. D'une manière générale, la présente invention n'est pas limitée aux exemples illustrés et/ou décrits ci-dessus.

L'étendue de la protection conférée est déterminée par les revendications annexées. Toutefois, la description et les dessins servent à interpréter les revendications.

L'usage des verbes « comprendre », « inclure », « comporter », ou toute autre variante, ainsi que leurs conjugaisons, ne peut en aucune façon exclure la présence d'éléments autres que ceux mentionnés. L'usage de l'article indéfini « un », « une », ou de l'article défini « le », « la » ou « l' », pour introduire un élément n'exclut pas la présence d'une pluralité de ces éléments. Les numéros de référence dans les revendications ne limitent pas leur portée.

## Revendications

1. Capteur (1) pour détecter des particules (5) dans un fluide d'un système de lubrification et comprenant :
- une unité de mesure (2) comprenant :
o une première bobine émettrice (21),
o une première bobine de réception (22) :
- couplée magnétiquement à, et
- coaxiale avec ladite première bobine émettrice (21);
- une unité de référence (3) comprenant :
o une deuxième bobine émettrice (31) identique à la première bobine émettrice (21),
o une deuxième bobine de réception (32) identique à la première bobine de réception (22):
- couplée magnétiquement à, et
- coaxiale avec ladite deuxième bobine émettrice (31);
les unités de mesure (2) et de référence (3) étant agencées de manière symétrique par rapport à un plan (6) situé entre l'unité de mesure (2) et l'unité de référence (3);
**caractérisé en ce qu'**il comprend en outre:
- un aimant (10) situé entre l'unité de mesure (2) et l'unité de référence (3) et à une même distance de l'unité de mesure (2) et de l'unité de référence (3) et agencé pour générer un champ magnétique continu pour attirer des particules (5) dudit fluide à proximité de l'unité de mesure (2) ;
- une paroi externe de contact prévue pour être en contact avec le fluide et pour empêcher l'entrée du fluide et des particules dans le capteur (1) ; et
l'unité de mesure (2) du capteur (1) étant située entre ladite paroi et l'unité de référence (3), de sorte que lesdites particules (5) soient maintenues à une plus grande distance de ladite unité de référence (3) que de ladite unité de mesure (2).

2. Capteur (1) selon la revendication précédente, **caractérisé en ce qu'**il comprend en outre un premier élément de carcasse ferromagnétique (23) traversant au moins partiellement les premières bobines émettrice (21) et de réception (22) et un deuxième élément de carcasse ferromagnétique (33) traversant au moins partiellement les deuxièmes bobines émettrice (31) et de réception (32).

3. Capteur (1) selon l'une quelconque des revendications précédentes, **caractérisé en ce que** l'aimant (10) est fait dans un matériau comprenant du samarium-cobalt.

4. Capteur (1) selon l'une quelconque des revendications précédentes, **caractérisé en ce qu'**il comprend en outre un premier circuit magnétique (17) entourant au moins partiellement ladite unité de mesure (2) pour concentrer des lignes de champ magnétiques générées par ledit aimant (10).

5. Capteur (1) selon l'une quelconque des revendications précédentes, **caractérisé en ce qu'**il comprend en outre un deuxième circuit magnétique (18) entourant au moins partiellement ladite unité de référence (3) pour concentrer des lignes de champ magnétiques générées par ledit aimant (10).

6. Capteur (1) selon l'une quelconque des revendications précédentes, **caractérisé en ce qu'**il comprend en outre un circuit électronique (40) agencé pour :
• envoyer un signal électrique source vers les première (21) et deuxième (31) bobines émettrices, et
• recevoir et amplifier un signal électrique de différence représentatif d'une différence entre une grandeur électrique mesurée aux bornes de la première bobine de réception (22) et une autre mesurée aux bornes de la deuxième bobine de réception (32).

7. Capteur (1) selon la revendication précédente, **caractérisé en ce que** le signal électrique source comprend des impulsions en courant et/ou en tension dont la durée est inférieure à 20% d'une durée séparant deux impulsions successives.

8. Capteur (1) selon l'une quelconque des revendications 6 ou 7, **caractérisé en ce que** le circuit électronique (40) comprend un circuit astable (101) pour créer un signal électrique comprenant des impulsions, un circuit d'inversion pour inverser ledit signal électrique comprenant des impulsions et un amplificateur (110) pour amplifier ledit signal électrique comprenant des impulsions inversé, afin de générer le signal électrique source.

9. Système de lubrification, en particulier d'une turbomachine, comportant un capteur (1) selon l'une quelconque des revendications précédentes.

10. Système de lubrification selon la revendication précédente, comprenant en outre un conduit (4) comprenant une partie de détection (80), le capteur (1) étant positionné de façon à ce que l'unité de mesure (2) se trouve entre la partie de détection (80) et l'unité de référence (3).

11. Turbomachine d'un aéronef comprenant un capteur (1) selon l'une quelconque des revendications 1 à 8.

12. Aéronef comprenant au moins un capteur (1) selon l'une quelconque des revendications 1 à 8.

13. Méthode de détection de particules (5) dans un fluide d'un système de lubrification, la méthode comprenant les étapes de:
• fournir un capteur (1) selon l'une quelconque des revendications 1 à 8,
• disposer le capteur (1) de façon à ce que des particules (5) présentes dans le fluide puissent influencer une induction magnétique captée par la première bobine de réception (22),
• envoyer un signal électrique source vers les première (21) et deuxième (31) bobines émettrices,
• amplifier une grandeur électrique de différence entre un signal électrique aux bornes de la première bobine de réception (22) et un signal électrique aux bornes de la deuxième bobine de réception (32).

## Patentansprüche

1. Sensor (1) zum Detektieren von Teilchen (5) in einem Fluid eines Schmiersystems und umfassend:
- eine Messeinheit (2), umfassend:
∘ eine erste Sendespule (21),
∘ eine erste Empfangsspule (22):
▪ magnetisch gekoppelt mit und
▪ koaxial mit der ersten Sendespule (21);
- eine Referenzeinheit (3), umfassend:
∘ eine zweite Sendespule (31), identisch mit der ersten Sendespule (21),
∘ eine zweite Empfangsspule (32), identisch mit der ersten Empfangsspule (22):
▪ magnetisch gekoppelt mit und
▪ koaxial mit der zweiten Sendespule (31);
wobei die Mess- (2) und Referenzeinheiten (3) bezogen auf eine Ebene (6), die zwischen der Messeinheit (2) und der Referenzeinheit (3) situiert ist, auf symmetrische Weise angeordnet sind;
**dadurch gekennzeichnet, dass** sie des Weiteren umfasst:
- einen Magneten (10), der zwischen der Messeinheit (2) und der Referenzeinheit (3) und in einem gleichen Abstand von der Messeinheit (2) und der Referenzeinheit (3) situiert und angeordnet ist, um ein kontinuierliches Magnetfeld zu generieren, um Teilchen (5) des Fluids in der Nähe der Messeinheit (2) anzuziehen;
- eine Kontaktaußenwand, vorgesehen, um mit dem Fluid in Kontakt zu sein, und um den Eintritt des Fluids und der Teilchen in den Sensor (1) zu verhindern; und
die Messeinheit (2) des Sensors (1) derart zwischen der Wand und der Referenzeinheit (3) situiert ist, dass die Teilchen (5) bei einem größeren Abstand von der Referenzeinheit (3) als von der Messeinheit (2) gehalten werden.

2. Sensor (1) nach dem vorstehenden Anspruch, **dadurch gekennzeichnet, dass** er des Weiteren ein erstes ferromagnetisches Karkassenelement (23), dass die erste Sende- (21) und Empfangsspule (22) mindestens teilweise durchquert, und ein zweites ferromagnetisches Karkassenelement (33) umfasst, dass die zweite Sende- (31) und Empfangsspule (32) mindestens teilweise durchquert.

3. Sensor (1) nach einem der vorstehenden Ansprüche, **dadurch gekennzeichnet, dass** der Magnet (10) aus einem Material hergestellt ist, umfassend Samarium-Cobalt.

4. Sensor (1) nach einem der vorstehenden Ansprüche, **dadurch gekennzeichnet, dass** er des Weiteren einen ersten Magnetkreis (17) umfasst, der die Messeinheit (2) mindestens teilweise umgibt, um die durch den Magneten (10) generierten Magnetfeldlinien zu konzentrieren.

5. Sensor (1) nach einem der vorstehenden Ansprüche, **dadurch gekennzeichnet, dass** er des Weiteren einen zweiten Magnetkreis (18) umfasst, der die Referenzeinheit (3) mindestens teilweise umgibt, um die durch den Magneten (10) erzeugten Magnetfeldlinien zu konzentrieren.

6. Sensor (1) nach einem der vorstehenden Ansprüche, **dadurch gekennzeichnet, dass** er des Weiteren einen elektronischen Schaltkreis (40) umfasst, angeordnet, um:
- ein elektrisches Quellensignal in Richtung der ersten (21) und zweiten (31) Sendespule zu schicken und
- ein elektrisches Differenzsignal zu empfangen und zu verstärken, das für einen Unterschied zwischen einer an Klemmen der ersten Empfangsspule (22) gemessenen elektrischen Größe und einer anderen an den Klemmen der zweiten Empfangsspule (32) gemessenen repräsentativ ist.

7. Sensor (1) nach dem vorstehenden Anspruch, **dadurch gekennzeichnet, dass** das elektrische Quellensignal Strom- und/oder Spannungsimpulse umfasst, von denen die Dauer weniger als 20% einer Dauer beträgt, die zwei aufeinander folgende Impulse trennt.

8. Sensor (1) nach einem der Ansprüche 6 oder 7, **dadurch gekennzeichnet, dass** der elektronische Schaltkreis (40) einen astabilen Schaltkreis (101), um ein Impulse umfassendes elektrisches Signal zu erzeugen, einen Inversionsschaltkreis, um das Impulse umfassende elektrische Signal zu invertieren, und einen Verstärker (110) umfasst, um das invertierte Impulse umfassende elektrische Signal zu verstärken, um das elektrische Quellensignal zu generieren.

9. Schmiersystem, insbesondere einer Turbomaschine, beinhaltend einen Sensor (1) nach einem der vorstehenden Ansprüche.

10. Schmiersystem nach dem vorstehenden Anspruch, umfassend des Weiteren eine Leitung (4), umfassend einen Detektionsteil (80), wobei der Sensor (1) auf eine Art positioniert ist, damit sich die Messeinheit (2) zwischen dem Detektionsteil (80) und der Referenzeinheit (3) befindet.

11. Turbomaschine eines Luftfahrzeugs, umfassend einen Sensor (1) nach einem der Ansprüche 1 bis 8.

12. Luftfahrzeug, umfassend mindestens einen Sensor (1) nach einem der Ansprüche 1 bis 8.

13. Verfahren zur Detektion von Teilchen (5) in einem Fluid eines Schmiersystems, wobei das Verfahren die Schritte umfasst:
- Bereitstellen eines Sensors (1) nach einem der Ansprüche 1 bis 8,
- Anbringen des Sensors (1) auf eine Art, damit die in dem Fluid vorhandenen Teilchen (5) eine durch die erste Empfangsspule (22) erfasste magnetische Induktion beeinflussen können,
- Schicken eines elektrischen Quellensignals in Richtung der ersten (21) und zweiten (31) Sendespule,
- Verstärken einer elektrischen Unterschiedsgröße zwischen einem elektrischen Signal an den Klemmen der ersten Empfangsspule (22) und einem elektrischen Signal an den Klemmen der zweiten Empfangsspule (32).

## Claims

1. Particle sensor (1) for detecting particles (5) in a fluid of a lubrication system, comprising:
- a measurement unit (2) comprising:
∘ a first transmitter coil (21),
∘ a first receiver coil (22):
▪ magnetically coupled to, and
▪ coaxial with, said first transmitter coil (21);
- a reference unit (3) comprising:
∘ a second transmitter coil (31) identical to the first transmitter coil (21),
∘ a second receiver coil (32) identical to the first receiver coil (22):
▪ magnetically coupled to, and
▪ coaxial with, said second transmitter coil (31);
the measurement (2) and reference (3) units being arranged symmetrically with respect to a plane (6) located between the measurement unit (2) and the reference unit (3);
**characterised in that** it further comprises:
- a magnet (10) which is located between the measurement unit (2) and the reference unit (3) and at the same distance from the measurement unit (2) and the reference unit (3) and arranged to generate a continuous magnetic field to attract particles (5) of said fluid in the vicinity of the measurement unit (2);
- an external contact wall provided so as to be in contact with the fluid and to prevent the fluid and the particles from entering the sensor (1); and
the measurement unit (2) of the sensor (1) being located between said wall and the reference unit (3), such that said particles (5) are kept at a greater distance from said reference unit (3) than from said measurement unit (2).

2. Sensor (1) according to the preceding claim, **characterised in that** it further comprises a first ferromagnetic casing element (23) passing at least partially through the first transmitter (21) and receiver (22) coils and a second ferromagnetic casing element (33) passing at least partially through the second transmitter (31) and receiver (32) coils.

3. Sensor (1) according to any of the preceding claims, **characterised in that** the magnet (10) is made of a material comprising samarium-cobalt.

4. Sensor (1) according to any of the preceding claims, **characterised in that** it further comprises a first magnetic circuit (17) at least partially surrounding said measurement unit (2) in order to concentrate magnetic field lines generated by said magnet (10).

5. Sensor (1) according to any of the preceding claims, **characterised in that** it further comprises a second magnetic circuit (18) at least partially surrounding said reference unit (3) in order to concentrate magnetic field lines generated by said magnet (10).

6. Sensor (1) according to any of the preceding claims, **characterised in that** it further comprises an electronic circuit (40) arranged to:
- send an electrical source signal to the first (21) and second (31) transmitter coils, and
- receive and amplify an electrical difference signal representative of a difference between an electrical quantity measured at the terminals of the first receiver coil (22) and another measured at the terminals of the second receiver coil (32).

7. Sensor (1) according to the preceding claim, **characterised in that** the electrical source signal comprises current and/or voltage pulses whose duration is less than 20% of a duration between two successive pulses.

8. Sensor (1) according to any of claims 6 or 7, **characterised in that** the electronic circuit (40) comprises an astable circuit (101) for creating an electrical signal comprising pulses, an inverting circuit for inverting said electrical signal comprising pulses, and an amplifier (110) for amplifying said inverted electrical signal comprising pulses, so as to generate the electrical source signal.

9. Lubrication system, in particular for a turbine engine, comprising a sensor (1) according to any of the preceding claims.

10. Lubrication system according to the preceding claim, further comprising a duct (4) comprising a detection portion (80), the sensor (1) being positioned such that the measurement unit (2) is located between the detection portion (80) and the reference unit (3).

11. Aircraft turbine engine comprising a sensor (1) according to any of claims 1 to 8.

12. Aircraft comprising at least one sensor (1) according to any of claims 1 to 8.

13. Method for detecting particles (5) in a fluid of a lubrication system, the method comprising the steps of:
- providing a sensor (1) according to any of claims 1 to 8,
- arranging the sensor (1) in such a way that particles (5) present in the fluid can influence a magnetic induction sensed by the first receiver coil (22),
- sending an electrical source signal to the first (21) and second (31) transmitter coils,
- amplifying an electrical quantity relating to the difference between an electrical signal at the terminals of the first receiver coil (22) and an electrical signal at the terminals of the second receiver coil (32).
